# EUROPÄISCHE PATENTSCHRIFT

(11) **EP 0 641 775 B1**
(45) Veröffentlichungstag und Bekanntmachung des Hinweises auf die Patenterteilung: **15.04.1998**
(21) Anmeldenummer: 94113811.7
(22) Anmeldetag: 02.09.1994
(51) Int. Cl.: C07C 319/02, C07D 333/38, C07C 323/51

(54) **Verfahren zur Herstellung von gamma-Mercaptocarbonsäurederivaten**
Process for the production of gamma-mercapto carbonic acid derivatives
Procédé pour la préparation des dérivés d'acide gamma mercapto carbonique

(30) Priorität: 03.09.1993 CH 2626/93
(43) Veröffentlichungstag der Anmeldung: 08.03.1995
(73) Patentinhaber: LONZA AG, CH-3945 Gampel/Wallis (CH)
(72) Erfinder: Quittmann, Wilhelm, Dr., Visp (Kanton Wallis) (CH); McGarrity, John, Dr., Visp (Kanton Wallis) (CH)
(74) Vertreter: Weinhold, Peter, Dr.

(56) Entgegenhaltungen:
- DE-A- 3 801 479
- J. ORG. CHEM. Bd. 54 , 1989 Seiten 20 - 21 MING-DE WANG ET AL. 'Regiospecific Carbonylation and Ring Expansion of Thietanes and Oxetanes Catalyzed by Cobalt and/or Ruthenium carbonyls'
- ORGANOMETALLICS Bd. 11 , 1992 Seiten 3422 - 3426 R.D.ADAMS ET AL. 'Ring Opening and Carbonylation of 3,3-Dimethyldietane Ligands in Ruthenium Carbonyl Cluster Complexes'

## Beschreibung

Die vorliegende Erfindung betrifft ein Verfahren zur Herstellung von γ-Mercaptocarbonsäurederivaten aus γ-Lactonen sowie neue β-substituierte γ-Thiolactone als Zwischenprodukte.

γ-Mercaptocarbonsäuren und deren Derivate wie Ester und Amide sind Zwischenprodukte beispielsweise für die Synthese von Leukotrien-Antagonisten (EP-A 0 480 717).
Die bekannten Verfahren zur Herstellung von γ-Mercaptocarbonsäurederivaten gehen beispielsweise von den entsprechenden γ-Halogencarbonsäurederivaten aus, deren Halogenatome durch nucleophile Substitution mit anorganischen Sulfiden bzw. Hydrogensulfiden gegen die Thiolgruppe ausgetauscht werden. Anstelle der Halogenverbindungen können auch Sulfonsäureester der entsprechenden Hydroxycarbonsäure eingesetzt werden, also beispielsweise die Mesylate oder Tosylate, welche auch mit organischen Schwefelverbindungen wie beispielsweise Thiocarbonsäuren bzw. deren Salzen zu den Mercaptoverbindungen umgesetzt werden können.

Alle diese Verfahren haben den Nachteil, dass zunächst ein "Hilfssubstituent" (Halogenid, Sulfonat) eingeführt werden muss, der anschliessend wieder abgespalten wird und letztlich als Abfall entsorgt werden muss.

Aufgabe der vorliegenden Erfindung war daher, ein Verfahren zur Herstellung von γ-Mercaptocarbonsäuren und deren Derivaten bereitzustellen, das von leicht zugänglichen Verbindungen ausgeht und wenig Abfall erzeugt.

Erfindungsgemäss wird diese Aufgabe durch das Verfahren nach Patentanspruch 1 gelöst.

Es wurde gefunden, dass sich die leicht zugänglichen γ-Lactone der allgemeinen Formel 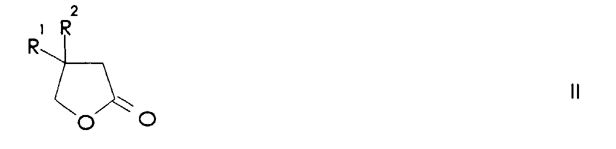 worin R¹ und R² entweder unabhängig voneinander Wasserstoff, C₁-C₆-Alkyl oder Aralkyl oder R¹ und R² zusammen eine -(CH₂)ₙ- Gruppe mit n = 2 bis 5 bedeuten, mit einem Thiocarboxylat der allgemeinen Formel 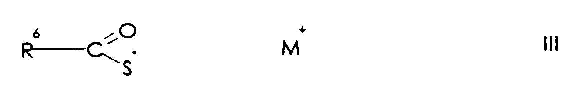 worin R⁶ eine C₁-C₆-Alkylgruppe oder eine Phenylgruppe und M ein Alkalimetall bedeutet, in die entsprechenden Thiolactone der allgemeinen Formel 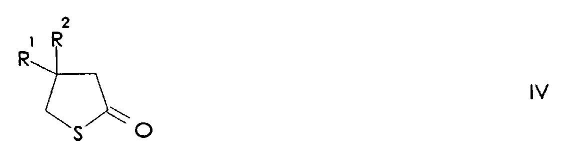 überführen lassen und diese durch Umsetzung mit einem Nucleophil der allgemeinen Formel

R³H V

worin R³ Hydroxy, C₁-C₆₋Alkoxy, Cycloalkyloxy, Aryloxy, Aralkyloxy oder NR⁴R⁵ bedeutet, wobei R⁴ und R⁵ unabhängig voneinander Wasserstoff, C₁-C₆-Alkyl, Cycloalkyl, Aryl oder Aralkyl sind oder R⁴ und R⁵ zusammen eine -(CH₂)₄-, -(CH₂)₅- oder -(CH₂)₂-O-(CH₂)₂-Kette bilden,
oder dem entsprechenden Anion die gewünschte γ-Mercaptocarbonsäure der allgemeinen Formel 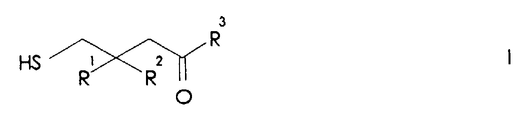 bzw. deren Ester, ein Amid oder ein Salz dieser Säure liefern.

Unter C₁-C₆-Alkyl sind hier jeweils nicht nur geradkettige primäre Alkylgruppen, also Methyl, Ethyl, Propyl, Butyl, Pentyl oder Hexyl, sondern auch alle isomeren sekundären, tertiären oder verzweigten Alkylgruppen mit bis zu 6 Kohlenstoffatomen zu verstehen, also beispielsweise auch Isopropyl, sec-Butyl, tert-Butyl, Isobutyl oder Isopentyl. Entsprechendes gilt für die Alkylkomponente der hier als C₁-C₆-Alkoxy bezeichneten Gruppen. Unter Cycloalkyl sind insbesondere Gruppen mit 3 - 6 Ringgliedern zu verstehen, also beispielsweise Cyclopropyl, Cyclobutyl, Cyclopentyl oder Cyclohexyl. Unter Aryl sind sowohl unsubstituierte als auch substituierte aromatische Reste zu verstehen, also beispielsweise Phenyl, Naphthyl, Chlorphenyl, Tolyl, Xylyl oder Methoxyphenyl und zwar jeweils in allen möglichen stellungsisomeren Formen.
Unter Aralkyl sind arylsubstituierte C₁-C₆-Alkylgruppen, also insbesondere Gruppen wie Benzyl, 1-Phenylethyl, 2-Phenylethyl oder 3-Phenylpropyl zu verstehen.

Die γ-Lactone der allgemeinen Formel II sind entweder kommerziell erhältlich (γ-Butyrolacton, R¹ = R² = H) oder nach bekannten Methoden herstellbar (z. B. β,β-Dimethyl-γ-butyrolacton, EP-A 172 371; für ein allgemein anwendbares Verfahren zur Herstellung von optisch aktiven β-Alkyl-γ-butyrolactonen siehe auch S. S. Canan Koch und A. R. Chamberlin, J. Org. Chem. 1993, *58*, 2725 - 2737).

Als Thiocarboxylat (III) eignen sich die Alkalisalze der Monothioalkansäuren, bei denen eine C1-C6-Alkylgruppe die Thiocarboxylatgruppe trägt, sowie die Alkalisalze der Thiobenzoesäure. Vorzugsweise wird ein Kaliumsalz eingesetzt, da dieses in organischen Lösungsmitteln besser löslich ist als die entsprechende Natriumverbindung.
Besonders bevorzugt als Thiocarboxylat (III) ist das Kaliumthioacetat. Die Umsetzung des Lactons mit dem Thiocarboxylat wird vorteilhaft in einem polaren aprotischen organischen Lösungsmittel wie beispielsweise Dimethylsulfoxid, N-Methylpyrrolidon, Sulfolan (Tetramethylensulfon), N,N-Dimethylformamid, N,N-Dimethylacetamid oder tetraalkylierte Harnstoffe wie beispielsweise 1,3-Dimethyl-3,4,5,6-tetrahydro-2(1H)-pyrimidinon (DMPU) durchgeführt. Besonders bevorzugt als Lösungsmittel ist N,N-Dimethylacetamid.

Die Reaktion wird zweckmässig bei erhöhter Temperatur, vorzugsweise bei 120 bis 170°C, durchgeführt.

Das Thiolacton (IV) kann auf übliche Weise durch Wasserzugabe und Extraktion mit einem unpolaren Lösungsmittel wie beispielsweise Dichlormethan von dem im Reaktionsgemisch vorhandenen Salz abgetrennt und durch fraktionierte Destillation oder einfaches Abdestillieren des Lösungsmittels isoliert werden.

Das Thiolacton (IV) wird anschliessend mit einem Nucleophil (V) umgesetzt, wobei der Lactonring geöffnet und je nach eingesetztem Nucleophil die γ-Mercaptocarbonsäure (I, R³ = OH) oder ein Ester oder Amid gebildet wird.

Als Nucleophile (V) eignen sich gemäss der obenstehenden Definition von R³ Wasser bwz. OH⁻ aus starken Basen wie Alkali- oder Erdalkalihydroxiden, also beispielsweise LiOH, NaOH, KOH, Ca(OH)₂, Ba(OH)₂ oder quartären Ammoniumhydroxiden. Vorzugsweise werden Alkalihydroxide eingesetzt. Bei der Umsetzung mit einer starken Base bildet sich das entsprechende Salz der γ-Mercaptocarbonsäure. Dieses kann als solches isoliert oder durch Zugabe einer starken Säure in die freie γ-Mercaptocarbonsäure übergeführt werden. Wird die starke Base im Überschuss eingesetzt, so kann auch das Dianion der γ-Mercaptocarbonsäure (mit deprotonierter Mercaptogruppe) gebildet werden.
Weiterhin eignen sich aliphatische (R³ = C₁-C₆-Alkoxy) und alicyclische (R³ = Cycloalkyloxy)-Alkohole wie beispielsweise Methanol, Ethanol, Propanol, Butanol, Pentanol, Hexanol, Isopropanol, sec-Butanol, tert-Butanol, Isobutanol, Isopentanol, Cyclopentanol oder Cyclohexanol. Vorzugsweise werden C₁-C₆-Alkanole bzw. die entsprechenden Alkoxide eingesetzt. Ebenfalls geeignet sind Phenole (R³ = Aryloxy) wie beispielsweise Phenol, Naphthole Chlorphenole, Kresole oder Xylenole oder Arylalkanole (R³ = Aralkoxy) wie beispielsweise Benzylalkohol, Phenethylalkohol oder 3-Phenyl-1-propanol.
Eine weitere Klasse von Nucleophilen (V), die im erfindungsgemässen Verfahren eingesetzt werden können, sind Stickstoffbasen, und zwar Ammoniak (R³ = NH2), primäre Amine (R³ = NHR⁴) und sekundäre Amine (R³ = NR⁴R⁵).
Als primäre Amine eignen sich sowohl Alkylamine (R⁴ = C₁-C₆-Alkyl) wie beispielsweise Methylamin, Ethylamin, Propylamin, Butylamin oder Isopropylamin als auch Cycloalkylamine (R⁴ = Cycloalkyl) wie beispielsweise Cyclohexylamin, aromatische Amine (R⁴ = Aryl) wie beispielsweise Anilin oder am Phenylring substituierte Aniline, oder Aralkylamine wie beispielsweise Benzylamin oder Phenylethylamine.
Als sekundäre Amine eignen sich solche mit beliebigen Kombinationen der vorstehend unter den primären Aminen aufgezählten Substituenten am Stickstoff, also beispielsweise Dialkylamine, Dicycloalkylamine, Aryl-alkylamine, Diarylamine sowie cyclische Amine wie Pyrrolidin (R⁴, R⁵ = -(CH₂)₄-), Piperidin (R⁴, R⁵ = -(CH₂)₅-) oder Morpholin (R⁴, R⁵ = -(CH₂)₂-O-(CH₂)₂-). Bevorzugte Stickstoffbasen sind Ammoniak und primäre Amine aus der Gruppe der C₁-C₆-Alkylamine, der Cycloalkylamine, der Arylamine oder der Aralkylamine.

Die Umsetzung kann unter basischen Bedingungen erfolgen, wobei gegebenenfalls das Anion des eingesetzten Nucleophils, also ein Hydroxid-, Alkoxid- oder Amidion reagiert. Beispielsweise kann mit einem Alkalimetallalkoholat im entsprechenden Alkohol (z.B. Natriummethylat in Methanol) der entsprechende Ester hergestellt werden.

Die Umsetzung mit Wasser oder Alkoholen kann auch unter Säurekatalyse erfolgen, so wird beispielsweise mit BF₃ in Methanol der Methylester erhalten.

Die nachfolgenden Beispiele verdeutlichen die Durchführung des erfindungsgemässen Verfahrens und die Herstellung der erfindungsgemässen Thiolactone.

### Beispiel 1

### 5-Thiaspiro[2.4]heptan-6-on (IV, R¹,R² = -(CH₂)₂-)

In einem 500 ml 4-Halskolben mit mechanischem Rührer, Luftkühler und Innenthermometer wurden unter Schutzgasatmosphäre 41,23 g 5-Oxaspiro[2.4]heptan-6-on (II, R¹, R² = -(CH₂)₂-; hergestellt nach EP 480 717, "Method N"), 0,40 g Hydrochinon und 190,0 g N,N-Dimethylacetamid vorgelegt. Nach Aufheizen des Kolbeninhalts auf 155°C wurden 50,4 g Kaliumthioacetat (Gehalt >99%) zugegeben. Das Gemisch wurde bei 155 ± 1°C 5 h gerührt. Nach dieser Zeit war der Umsatz (nach GC) praktisch quantitativ. Das Reaktionsgemisch wurde auf Raumtemperatur abgekühlt und zuerst mit 2,3 g konzentrierter Essigsäure und anschliessend mit 185 ml Wasser versetzt.
Die Mischung wurde zur Entfernung der Salze 15 min bei Raumtemperatur gerührt, anschliessend wurden die Phasen im Scheidetrichter getrennt und die wässrige Phase noch zweimal mit je 95 ml Dichlormethan extrahiert.
Die vereinigten organischen Phasen wurden über Natriumsulfat getrocknet und am Rotationsverdampfer vom Dichlormethan befreit. Der Rückstand wurde bei 5,1 mbar über eine 30 cm-Füllkörperkolonne destilliert, wobei zunächst das N,N-Dimethylacetamid und anschliessend bei 76 - 78°C das Produkt mit einer Reinheit von 99,8% (GC) überging.
- Ausbeute:: 93,0% d. Th.
- ¹H-NMR (CDCl₃, 300 MHz): δ: 0,76 (m, 4H)
2,49 (s, 2H)
3,22 (s, 2H)
- IR (Film, cm⁻¹): 3001 (C-H); 1709 (ss, C=0); 1036.

### Beispiel 2

### [1-(Mercaptomethyl)cyclopropyl]essigsäure (I, R¹,R² = -(CH₂)₂-, R³ = OH)

Bei Raumtemperatur wurden 9,0 g (0,07 mol) 5-Thiaspiro[2.4]heptan-6-on (IV, R¹,R² = -(CH₂)₂-; hergestellt nach Beispiel 1) unter Schutzgas in einer Portion zu einer Lösung von 3,7 g (91 mmol) NaOH in 55 ml Wasser hinzugefügt. Das Gemisch wurde 2,5 h zum Rückfluss erhitzt und anschliessend auf <10°C abgekühlt. Bei dieser Temperatur wurden innerhalb von 5 min 15 ml 6,07 n Salzsäure zugetropft, wobei ein weisser Feststoff ausfiel. Dieser wurde durch Zugabe von 18 ml Methyl-tert-butylether in Lösung gebracht.
Die Phasen wurden getrennt und die wässrige Phase noch zweimal mit je 36 ml Methyl-tert-butylether extrahiert. Die vereinigten organischen Phasen wurden durch azeotrope Destillation entwässert, wobei im Verlauf der Destillation noch weitere 10 ml des Ethers zugegeben wurden.
Nach vollständiger Abdestillation wurden die letzten Spuren von Ether und Wasser im Vakuum bei 7,5 mbar und 62°C entfernt.
Nach dem Abkühlen wurde das Vakuum mit Inertgas aufgehoben.
Die γ-Mercaptosäure wurde als farbloser kristalliner Feststoff erhalten, der wegen seiner Oxidationsempfindlichkeit unter Inertgas aufbewahrt werden muss.
- Ausbeute:: 9,8 g,entspr. 95% d. Th.
- Fp.:: 42,5 - 43,8°C
- ¹H-NMR (CDCl₃, 300 MHz): δ: 0,55 - 0,68 (m, 4H)
1,38 (t, 1H)
2,54 (s, 2H)
2,65 (d, 2H)
- IR (Film auf NaCl, cm⁻¹): 3077,4, 3037,6 (C-H); 1705,5 (ss, C=0):
2570 (m, S-H).

Zur weiteren Charakterisierung wurde das entsprechende Disulfid hergestellt: [1-(1-Carboxymethylcyclopropylmethyldisulfanylmethyl)cyclopropyl]essigsäure 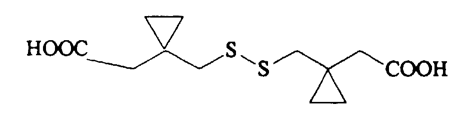 Analog zur vorstehenden Vorschrift wurden 4,6 g (34,4 mmol) 5-Thiaspiro[2.4]heptan-6-on (95,5%ig) mit 1,8 g (44,7 mmol) Natriumhydroxid hydrolysiert. In die auf 20 - 25°C abgekühlte Reaktionsmischung wurde in kleinen Portionen eine Lösung von 6,0 g Kaliumiodid und 4,4 g lod in 20 ml Wasser gegeben, so dass die Suspension gegen Ende der Zugabe noch leicht braun gefärbt war. Die Suspension wurde noch ca. 30 min bei Raumtemperatur gerührt und anschliessend mit wenigen Tropfen wässriger Natriumpyrosulfit-Lösung entfärbt. Das Reaktionsgemisch wurde mit 200 ml Diethylether extrahiert. Die organische Phase wurde über Natriumsulfat getrocknet, filtriert und am Rotationsverdampfer eingeengt. Der zurückbleibende weisse Feststoff wurde im Wasserstrahlvakuum bei 60°C getrocknet und aus Essigsäureethylester umkristallisiert.
- Fp.:: 135,5- 136,2°C
- ¹H-NMR (DMSO-d₆, 400 MHz): δ: 0,6 (m, 8H)
2,3 (s, 4H)
2,9 (s, 4H)
12,05 (br. s, 2H)

### Beispiel 3

### 4,4-Dimethyldihydrothiophen-2-on (IV, R¹ = R² = CH₃)

Analog zu Beispiel 1 wurden 2,6 g 4,4-Dimethyldihydro-2(3H)-furanon (II, R¹ = R² = CH₃) in Gegenwart von 22 mg Hydrochinon in 5,0 g N,N-Dimethylacetamid bei 160°C mit 3,1 g Kaliumthioacetat umgesetzt. Nach 7 h Reaktionszeit betrug der Umsatz 98,5% (GC).
Nach Aufarbeitung analog zu Beispiel 1 und Destillation über eine kleine Kolonne wurde das Thiolacton in einer Ausbeute von 88,3% d. Th. mit einer Reinheit von 96,7% (GC) erhalten.
- ¹H-NMR (CDCl₃):δ: 1,27 (s, 6H)
2,40 (s, 2H)
3,19(S, 2H)

### Beispiel 4

### 3,3-Dimethyl-4-mercaptobuttersäuremethylester (I, R¹ = R² = CH₃, R³ = OCH₃)

In 5 ml Methanol wurden 4,0 g 4,4-Dimethyldihydrothiophen-2-on (96,7%ig, hergestellt nach Beispiel 3) gelöst, unter Feuchtigkeitsausschluss mit 6,5 g einer 25%igen methanolischen Natriummethylatlösung versetzt und anschliessend 2,5 h zum Rückfluss erhitzt. Anschliessend wurde das Methanol im Vakuum am Rotationsverdampfer abdestilliert und der Rückstand mit 2 ml Wasser und 1,8 g Essigsäure versetzt. Das so erhaltene Gemisch wurde mit 10 ml Dichlormethan extrahiert und der Extrakt mit Natriumsulfat getrocknet. Das durch Abdestillieren des Dichlormethans erhältliche Rohprodukt enthielt 83,4% der Titelsubstanz neben 6,7% Edukt (GC).
- ¹H-NMR (CDCl₃):δ: 1,07 (s, 6H)
1,45 (t, 1H)
2,46 (S, 2H)
2,59 (d, 2H)
3,68 (s, 3H).

### Beispiel 5

### [1-(Mercaptomethyl)cyclopropyl]essigsäure, Lithiumsalz

In einer Mischung aus 8,5 ml Wasser und 4,1 g Methanol wurden 0,63 g Lithiumhydroxid-Monohydrat gelöst und 1,9 g (15 mmol) 5-Thiaspiro[2.4]heptan-6-on (hergestellt nach Beispiel 1) hinzugefügt.
Das Gemisch wurde 3 h am Rückfluss gekocht, wobei die anfangs vorhandenen zwei Phasen in eine nahezu homogene Lösung übergingen.
Nachdem mittels GC kein restliches Edukt mehr nachweisbar war, wurde das Reaktionsgemisch bei 70°C in Wasserstrahlvakuum eingedampft und der feste weisse Rückstand bei dieser Temperatur im Vakuum getrocknet. Zur Reinigung wurde das getrocknete Produkt in 10 ml Dichlormethan suspendiert und nach 30 min Rühren bei Raumtemperatur abfiltriert. Nach Trocknung bei 40°C im Vakuum wurde ein weisses Kristallpulver erhalten.
- Ausbeute:: 1,9 g,entspr. 85% d. Th.
- ¹H-NMR (D₂O, int. Standard 3-(Trimethyl silyl) propionsäure-d₄ Natriumsalz):δ: 0,48 - 0,59 (m, 4H)
2,32 (s, 2H)
2,59 (s, 2H)
- ¹³C-NMR (D₂O):δ: 15,42
22,92
35,63
45,24
184,44
- Elementaranalyse (ICP): Gef.Li 5,22%
Ber.Li 4,56%

### Beispiel 6

### N-Benzyl-[1-(mercaptomethyl)cyclopropyl]acetamid (I, R¹,R² = -(CH₂)₂-, R³ = NHCH₂C₆H₅)

In 2,5 g Dioxan wurden 0,64 g (5 mmol) 5-Thiaspiro[2.4]heptan-6-on (hergestellt nach Beispiel 1) mit der äquimolaren Menge Benzylamin 22,5 h unter Schutzgas zum Rückfluss erhitzt. Anschliessend wurde das Dioxan im Vakuum abdestilliert. Das zurückbleibende farblose viskose Öl kristallisierte nach Zusatz von etwas Petrolether bei 4°C langsam zu feinen Nadeln.
- Ausbeute:: 1,1 g,entspr. 91% d. Th.
- ¹H-NMR (CDCl₃): δ: 0,52 - 0,68 (m, 4H)
1,40 (t, 1H)
2,37 (s, 2H)
2,59 (d, 2H)
4,45 (d, 2H)
6,18(br.s, 1H)
7,23-7,41 (m, 5H).

### Beispiel 7

### [1-(Mercaptomethyl)cyclopropyl]acetamid (1, R¹,R² = -(CH₂)₂-, R³ = NH₂)

In eine Lösung von 5 g (39 mmol) 5-Thiaspiro[2.4]heptan-6-on (hergestellt nach Beispiel 1) in 50 ml N,N-Dimethylacetamid wurde bei 50 - 52°C für 15 h ein leichter Ammoniakstrom eingeleitet. Aus dem gelben Reaktionsgemisch wurde im Vakuum das Lösungsmittel abdestilliert, wobei das Produkt auskristallisierte. Zur Reinigung wurde aus Acetonitril umkristallisiert, wobei fast farblose Kristalle erhalten wurden.
- Fp.:: 130- 133,2°C
- ¹H-NMR (CDCl₃): δ: 0,42 - 0,58 (m, 4H)
2,18 (s, 2H)
2,16 (t, 1H)
6,76 (br. s, 2H).

### Beispiel 8

### [1-(Mercaptomethyl)cyclopropyl]essigsäuremethylester (I, R¹,R² = -(CH₂)₂-, R³ = OCH₃)

In 5 ml Methanol wurden 1,2 g 5-Thiaspiro[2.4]heptan-6-on (hergestellt nach Beispiel 1) gelöst und mit 10 Tropfen einer 1,3 m Lösung von Bortrifluorid-Diethylether-Addukt versetzt.
Das Gemisch wurde unter Feuchtigkeitsausschluss 22 h im geschlossenen Rohr erhitzt und anschliessend über Nacht bei Raumtemperatur stehengelassen. Mittels GC wurde ein Umsatz von 94,2% bestimmt. Das Produkt wurde nicht isoliert, sondern in der Reaktionsmischung durch ¹H-NMR identifiziert.
- ¹H-NMR (CDCl₃):δ: 0,49 - 0,65 (m, 4H)
1,38 (t, 1H)
2,50 (s, 2H)
2,64 (d, 2H)
3,68 (s, 3H).

## Patentansprüche

1. Verfahren zur Herstellung von γ-Mercaptocarbonsäurederivaten der allgemeinen Formel 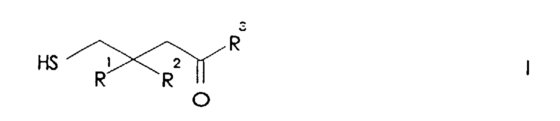
worin R¹ und R² entweder unabhängig voneinander Wasserstoff, C₁-C₆-Alkyl oder Aralkyl oder zusammen eine -(CH₂)ₙ- Gruppe mit n = 2 bis 5,
R³ Hydroxy, C₁-C₆-Alkoxy, Cycloalkyloxy, Aryloxy, Aralkyloxy oder -NR⁴R⁵ und R⁴ und R⁵ entweder unabhängig voneinander Wasserstoff, C₁-C₆-Alkyl, Cycloalkyl, Aryl oder Aralkyl oder R⁴ und R⁵ zusammen eine -(CH₂)₄-, -(CH₂)₅- oder -(CH₂)₂-O-(CH₂)₂-Gruppe bedeuten,
oder deren Salzen, dadurch gekennzeichnet, dass ein γ-Lacton der allgemeinen Formel 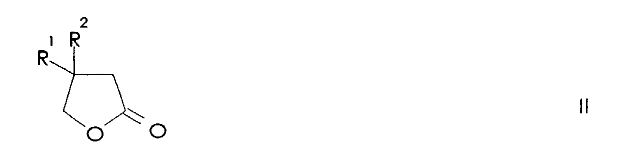 worin R¹ und R² die oben genannten Bedeutungen haben, mit einem Thiocarboxylat der allgemeinen Formel 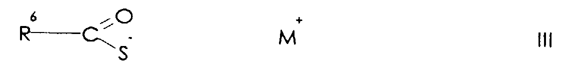 worin R⁶ eine C₁-C₆-Alkylgruppe oder eine Phenylgruppe und M ein Alkalimetall bedeutet, in einem polaren Lösungsmittel zu dem entsprechenden Thiolacton der allgemeinen Formel 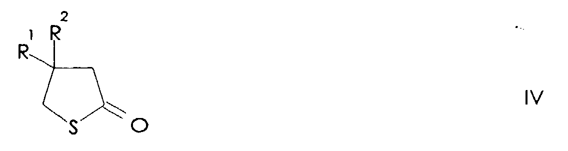 worin R¹ und R² die oben genannten Bedeutungen haben, umgesetzt und anschliessend das Thiolacton mit einem Nucleophil der allgemeinen Formel
R³H V
worin R³ die oben genannte Bedeutung hat, oder dem entsprechenden Anion
(R³)⁻ V'
zur Zielverbindung 1 oder einem entsprechenden Salz umgesetzt wird.

2. Verfahren nach Anspruch 1, dadurch gekennzeichnet, dass als polares Lösungsmittel für die Umsetzung des γ-Lactons mit dem Thiocarboxylat ein polares aprotisches Lösungsmittel aus der Gruppe bestehend aus Dimethylsulfoxid, Sulfolan, N-Methylpyrrolidon, N,N-Dimethylformamid, N,N-Dimethylacetamid und 1,3-Dimethyl-3,4,5,6-tetrahydro-2(1H)-pyrimidinon eingesetzt wird.

3. Verfahren nach Anspruch 2, dadurch gekennzeichnet, dass als polares Lösungsmittel für die Umsetzung des γ-Lactons mit dem Thiocarboxylat N,N-Dimethylacetamid eingesetzt wird und die Umsetzung bei 120 bis 170°C durchgeführt wird.

4. Verfahren nach einem der Ansprüche 1 bis 3, dadurch gekennzeichnet, dass als Thiocarboxylat Kaliumthioacetat eingesetzt wird.

5. Verfahren nach einem der Ansprüche 1 bis 4, dadurch gekennzeichnet, dass als Nucleophil R³H ein C₁-C₆-Alkanol in Gegenwart von Bortrifluorid als Katalysator eingesetzt wird.

6. Verfahren nach einem der Ansprüche 1 bis 4, dadurch gekennzeichnet, dass als Nucleophil (R³)⁻ das Hydroxidion aus einem Alkalihydroxid eingesetzt wird.

7. Verfahren nach einem der Ansprüche 1 bis 4, dadurch gekennzeichnet, dass als Nucleophil (R³)⁻ das Alkoxidion aus einem C₁-C₆-Alkanol eingesetzt wird.

8. Verfahren nach einem der Ansprüche 1 bis 4, dadurch gekennzeichnet, dass als Nucleophil R³H Ammoniak eingesetzt wird.

9. Verfahren nach einem der Ansprüche 1 bis 4, dadurch gekennzeichnet, dass als Nucleophil R³H ein Amin aus der Gruppe der C₁-C₆-Alkylamine, der Cycloalkylamine, der Arylamine oder Aralkylamine eingesetzt wird.

10. Thiolactone der Formel 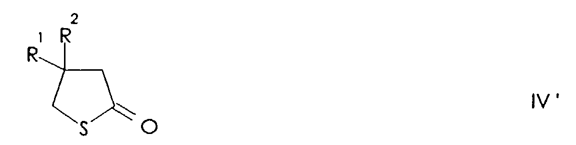 worin R¹ und R² die in Anspruch 1 genannten Bedeutungen haben, ausgenommen die Verbindungen Dihydrothiophen-2-on und 4,4-Dimethyldihydrothiophen-2-on.

11. 5-Thiaspiro[2.4]heptan-6-on der Formel

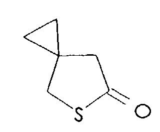

## Claims

1. A method for producing γ-mercaptocarboxylic acid derivatives having the general formula 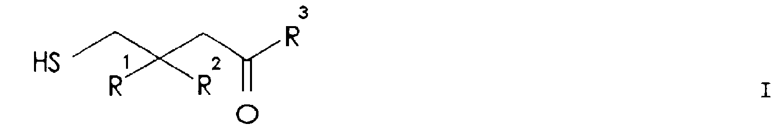
wherein R¹ and R² either independently signify hydrogen, C₁-C₆ alkyl or aralkyl, or jointly signify a -(CH₂)ₙ group with n=2 to 5,
R³ signifies hydroxy, C₁-C₆ alkoxy, cycloalkyloxy, aryloxy, aralkyloxy or -NR⁴R⁵, and
R⁴ and R⁵ either independently signify hydrogen, C₁-C₆ alkyl, cycloalkyl, aryl or aralkyl, or R⁴ and R⁵ jointly signify a -(CH₂)₄, -(CH₂)₅ or -(CH₂)₂-O-(CH₂)₂ group,
or salts thereof, characterized in that a γ-lactone having the general formula 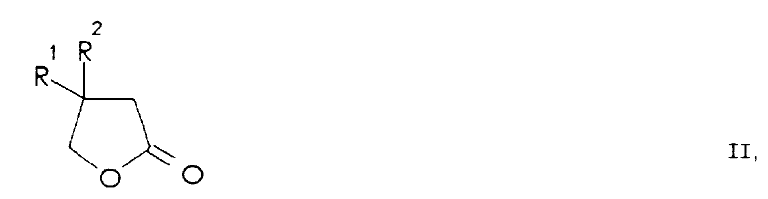 wherein R¹ and R² have the above specified meanings, is reacted in a polar solvent with a thiocarboxylate having the general formula 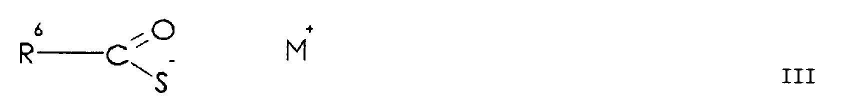 wherein R⁶ signifies a C₁-C₆ alkyl group or a phenyl group and M signifies an alkali metal, to form the corresponding thiolactone having the general formula 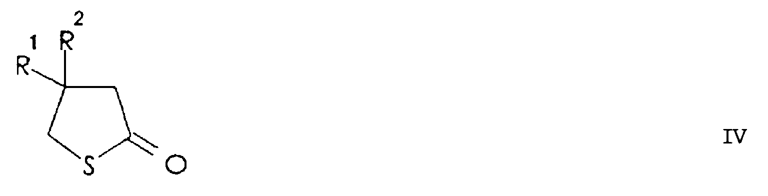 wherein R¹ and R² have the above specified meanings, and subsequently said thiolactone is reacted with a nucleophile having the general formula
R³H V
wherein R³ has the above specified meaning, or with the corresponding anion
(R³)⁻ V'
to form target compoound I or a corresponding salt.

2. The method according to claim 1, characterized in that a polar aprotic solvent from among the group consisting of dimethyl sulfoxide, sulfolane, N-methylpyrrolidone, N,N-dimethylformamide, N,N-dimethylacetamide and 1,3-dimethyl-3,4,5,6-tetrahydro-2(1H)-pyrimidinone is used as said polar solvent for reacting said γ-lactone with said thiocarboxylate.

3. The method according to claim 2, characterized in that N,N-dimethylacetamide is used as said polar solvent for reacting said γ-lactone with said thiocarboxylate, and that the reaction is performed at 120°C to 170°C.

4. The method according to any one of claims 1 to 3, characterized in that potassium thioacetate is used as said thiocarboxylate.

5. The method according to any one of claims 1 to 4, characterized in that a C₁-C₆ alkanol is utilized as said nucleophile R³H in the presence of boron trifluoride as a catalyst.

6. The method according to any one of claims 1 to 4, characterized in that the hydroxide ion from an alkali hydroxide is utilized as said nucleophile (R³)⁻.

7. The method according to any one of claims 1 to 4, characterized in that the alkoxide ion from a C₁-C₆ alkanol is utilized as said nucleophile (R³)⁻.

8. The method according to any one of claims 1 to 4, characterized in that ammonia is utilized as said nucleophile R³H.

9. The method according to any one of claims 1 to 4, characterized in that an amine from among the group consisting of the C₁-C₆ alkylamines, the cycloalkylamines, the arylamines or aralkylamines is utilized as said nucleophile R³H.

10. Thiolactones having the formula 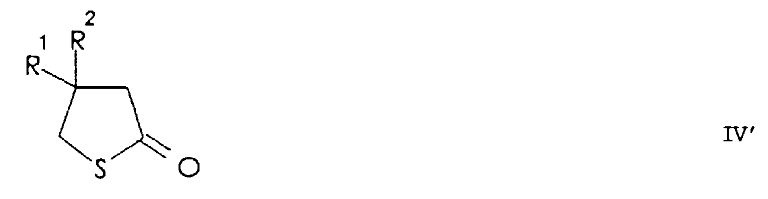 wherein R¹ and R² have the meanings specified in claim 1 with the exception of the compounds dihydrothiophen-2-one and 4,4-dimethyldihydrothiophen-2-one.

11. 5-thiaspiro[2.4]heptan-6-one having the formula

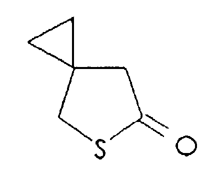

## Revendications

1. Procédé de préparation de dérivés d'acides γ-mercaptocarboxyliques de formule générale: 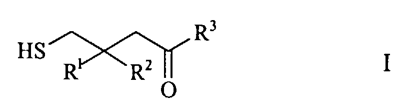 dans laquelle
R¹ et R² sont, indépendamment l'un de l'autre, un atome d'hydrogène ou un groupe alkyle en C₁-C₆ ou aralkyle, ou forment ensemble un groupe -(CH₂)ₙ- où n = 2 à 5,
R³ représente un groupe hydroxy, alcoxy en C₁-C₆, cycloalkyloxy, aryloxy, aralkyloxy ou -NR⁴R⁵ et R⁴ et R⁵ sont indépendamment l'un de l'autre un atome d'hydrogène ou un groupe alkyle en C₁-C₆, cycloalkyle, aryle ou aralkyle, ou bien R⁴ et R⁵ forment ensemble un groupe -(CH₂)₄-, -(CH₂)₅- ou -(CH₂)₂-O-(CH₂)₂-,
ou de leurs sels, caractérisé en ce que l'on fait réagir une γ-lactone de formule générale 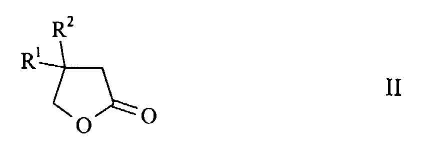 dans laquelle R¹ et R² ont la signification indiquée ci-dessus, avec un thiocarboxylate de formule générale 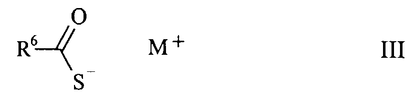 dans laquelle R⁶ représente un groupe alkyle en C₁-C₆ ou un groupe phényle et M est un métal alcalin, dans un solvant polaire, pour former la thiolactone correspondante de formule générale 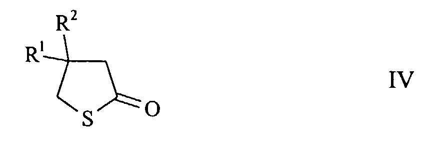 dans laquelle R¹ et R² ont la signification indiquée ci-dessus, puis on fait réagir la thiolactone avec un nucléophile de formule générale
R³H V
dans laquelle R³ a la signification indiquée ci-dessus, ou avec l'anion correspondant
(R³)⁻ V'
pour former le composé désiré I ou un sel correspondant.

2. Procédé selon la revendication 1, caractérisé en ce que l'on utilise comme solvant polaire pour la réaction de la γ-lactone avec le thiocarboxylate un solvant aprotique polaire du groupe constitué par le diméthylsulfoxyde, le sulfolane, la N-méthylpyrrolidone, le N,N-diméthylformamide, le N,N-diméthylacétamide et la 1,3-diméthyl-3,4,5,6-tétrahydro-2(1H)-pyrimidinone.

3. Procédé selon la revendication 2, caractérisé en ce que l'on utilise comme solvant polaire pour la réaction de la γ-lactone avec le thiocarboxylate le N,N-diméthylacétamide et on effectue la réaction à une température de 120 à 170°C.

4. Procédé selon l'une des revendications 1 à 3, caractérisé en ce que l'on utilise comme thiocarboxylate le thioacétate de potassium.

5. Procédé selon l'une des revendications 1 à 4, caractérisé en ce que l'on utilise comme nucléophile R³H un alcanol en C₁-C₆ en présence de trifluorure de bore comme catalyseur.

6. Procédé selon l'une des revendications 1 à 4, caractérisé en ce que l'on utilise comme nucléophile (R³)⁻ l'ion hydroxyde d'un hydroxyde de métal alcalin.

7. Procédé selon l'une des revendications 1 à 4, caractérisé en ce que l'on utilise comme nucléophile (R³)⁻ l'ion alkylate d'un alcanol en C₁-C₆.

8. Procédé selon l'une des revendications 1 à 4, caractérisé en ce que l'on utilise de l'ammoniac comme nucléophile R³H.

9. Procédé selon l'une des revendications 1 à 4, caractérisé en ce que l'on utilise comme nucléophile R³H une amine du groupe des alkylamines en C₁-C₆, des cycloalkylamines, des arylamines ou des aralkylamines.

10. Thiolactones de formule 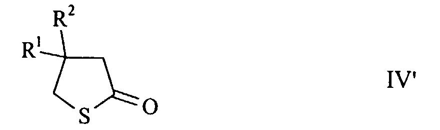 dans laquelle R¹ et R² ont la signification indiquée dans la revendication 1, à l'exception des composés dihydrothiophène-2-one et 4,4-diméthyldihydrothiophène-2-one.

11. 5-Thiaspiro[2.4]heptane-6-one de formule
